# EUROPEAN PATENT APPLICATION

(11) **EP 4 318 714 A1**
(43) Date of publication of application: **07.02.2024**
(21) Application number: 22937654.6
(22) Date of filing: 11.05.2022
(51) Int. Cl.: H01M 10/0567, H01M 10/0569

(54) **ELECTROLYTE, SECONDARY BATTERY, BATTERY MODULE, BATTERY PACK AND ELECTRICAL DEVICE**

(71) Applicant: Contemporary Amperex Technology Co., Limited, Ningde City, Fujian 352100 (CN)
(72) Inventor: PENG, Chang, Ningde City, Fujian 352100 (CN); CHEN, Peipei, Ningde City, Fujian 352100 (CN); ZHANG, Limei, Ningde City, Fujian 352100 (CN); WU, Degui, Ningde City, Fujian 352100 (CN)
(74) Representative: Herrmann, Uwe
(86) International application number: PCT/CN2022/092189
(87) International publication number: WO 2023/216130

(57) **Abstract**

The present disclosure relates to an electrolytic solution for a lithium-ion secondary battery including a solvent, additive, and lithium salt. The solvent includes carboxylic ester, and the additive includes fluorosulfonic acid lactone of Formula I in the description. In the electrolytic solution, a content W of carboxylic ester and a content a of fluorosulfonic acid lactone of Formula I satisfy 430.1 > 1000*a/W> 0.163, optionally 202.02 > 1000*a/w≥ 1.635, and further optionally 100.059 ≥ 1000*a/w ≥ 4.915, where a and W are both in wt% based on a weight of the electrolytic solution. The secondary battery containing the electrolytic solution has excellent quick charging performance and a long cycle life.

## Description

### TECHNICAL FIELD

The present disclosure relates to the technical field of lithium batteries, and in particular to an electrolytic solution, a secondary battery, a battery module, a battery pack, and a powered device.

### BACKGROUND

The lithium-ion battery becomes the most popular energy storage system due to characteristics of high working potential, long service life and environmental friendliness, and is widely applied to the fields of pure electric vehicles, hybrid electric vehicles, intelligent power grids and the like. With higher requirements of people for cruising ability, the mileage anxiety problem of people on the electric vehicle needs to be solved, and a lithium-ion battery system with a higher energy density is urgently needed to be developed.

The lithium iron phosphate (LFP) battery has high cycle life and safety performance, but the energy density of the lithium iron phosphate (LFP) battery is low as a fatal defect of the lithium iron phosphate (LFP) battery. In order to improve the energy density of the LFP battery, the coating weight of the positive electrode material is to be improved, which becomes the primary strategy of researchers. However, after the coating weight is improved, the thickness of the electrode plate is remarkably increased, and the corresponding lithium-ion transmission path is remarkably increased. Accordingly, the electrolytic solution of a common carbonate solvent system is difficult to meet the quick charging requirement of the existing thick coated LFP battery. The carboxylate solvent system is low in viscosity, and the corresponding electrolytic solution has high conductivity. However, since the compatibility of the carboxylate and the negative electrode of the carboxylate solvent system is poor, the battery cell of the LFP system has short cycle and the storage life. Therefore, the existing electrolytic solution system still needs to be improved.

### SUMMARY

In view of the above subject, the present disclosure is to provide an electrolytic solution for a lithium-ion secondary battery, and the electrolytic solution includes carboxylic ester and fluorosulfonic acid lactone with a specific structure, such that the corresponding lithium-ion battery has excellent quick charging performance and a long cycle life.

In order to achieve the above, a first aspect of the present disclosure provides an electrolytic solution for a lithium-ion secondary battery, which includes a solvent, additive, and lithium salt. The solvent includes carboxylic ester, and the additive includes fluorosulfonic acid lactone of Formula I as below 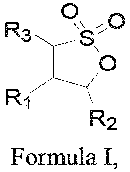 where R₁, R₂ and R₃ each are H or F independently, and at least one of R₁, R₂ and R₃ is F and at least one other of R₁, R₂ and R₃ is H. In the electrolytic solution, a content W of carboxylic ester and a content a of fluorosulfonic acid lactone of Formula I satisfy 430.1 > 1000*a/W > 0.163, optionally 202.02 > 1000*a/w ≥ 1.635, and further optionally 100.059 ≥ 1000*a/w ≥ 4.915, wherein a and W are both in wt% based on a weight of the electrolytic solution.

Therefore, the electrolytic solution contains carboxylic ester and fluorosulfonic acid lactone of Formula I at a specific proportion, such that compatibility of carboxylic ester solvent and a negative electrode can be adjusted, and reduction of carboxylic ester in the electrolytic solution is further prevented. Accordingly, the corresponding lithium-ion battery has excellent quick charging performance and a long cycle life.

In an embodiment, the content W of carboxylic ester is 30 wt% to 70 wt%, and the content a of fluorosulfonic acid lactone of Formula I is 0.01 wt% to 10 wt%, each based on the weight of the electrolytic solution. Therefore, the electrolytic solution contains the carboxylic ester and the fluorosulfonic acid lactone of Formula I with the above contents, such that the corresponding lithium-ion battery has excellent quick charging performance and a long cycle life.

In an embodiment, the carboxylic ester is at least one selected from methyl formate, methyl acetate, ethyl acetate, propyl acetate, methyl propionate, ethyl propionate, and propyl propionate, and fluorosulfonic acid lactone of Formula I is 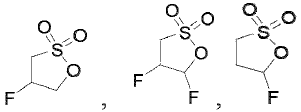 or

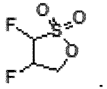

Therefore, the electrolytic solution contains fluorosulfonic acid lactone of the above type of Formula I, such that the corresponding lithium-ion battery has excellent quick charging performance and a long cycle life.

In an embodiment, in the electrolytic solution, the solvent is 70-90 wt%, the additive is 0.01-20 wt%, and lithium salt is 10-22 wt%. A sum of weight percentages of the solvent, the additive and lithium salt is 100% based on the weight of the electrolytic solution.

Therefore, the electrolytic solution contains the solvent, the additive, and lithium salt with the above contents, such that the corresponding lithium-ion battery has excellent quick charging performance and a long cycle life.

In an embodiment, in the electrolytic solution, a weight percentage of carboxylic ester in the solvent is 30% to 80% based on a weight of the solvent, and a weight percentage of the fluorosulfonic acid lactone in the additive is 20 wt% to 100 wt% based on a weight of the additive. Therefore, the electrolytic solution contains the carboxylic ester and the fluorosulfonic acid lactone of Formula I with the above contents, such that the corresponding lithium-ion battery has excellent quick charging performance and a long cycle life.

In an embodiment, the additive further inlcudes O=C=N-R-N=C=O additive, where R is one of substituted or unsubstituted C1-C6 alkylene or alkyleneoxy, substituted or unsubstituted C2-C6 alkenylene or alkenyloxy, and substituted or unsubstituted C6-C12 arylene; optionally at least one of hexamethylene diisocyanate (HDI), toluene diisocyanate (TDI), isophorone diisocyanate (IPDI), diphenylmethane diisocyanate (MDI), and dicyclohexylmethane diisocyanate (H12MDI); and further optionally hexamethylene diisocyanate or toluene diisocyanate. Therefore, the electrolytic solution further enables the corresponding lithium-ion battery to have excellent quick charging performance and a long cycle life by containing the above type of isocyanate.

In an embodiment, the solvent further includes a solvent of cyclic carbonate, and the cyclic carbonate is optionally one or more of ethyl propyl carbonate, propylene carbonate. A content of the cyclic carbonate is 8.5% to 35% of the electrolytic solution. Therefore, the electrolytic solution further enables the corresponding lithium-ion battery to have excellent quick charging performance and a long cycle life by containing the above type of solvent.

In an embodiment, the lithium salt is one or more selected from lithium hexafluorophosphate, lithium tetrafluoroborate, lithium perchlorate, lithium hexafluoroarsenate, lithium bis(fluorosulfonyl) imide, lithium bis(trifluoromethanesulfonyl) imide, lithium trifluoromethanesulfonate, lithium difluoro(oxalato)borate, lithium bis(oxalate) borate, lithium difluorophosphate, lithium difluorobis(oxalato) phosphate, and lithium tetrafluorooxalate phosphate, optionally one or both of lithium hexafluorophosphate or lithium bis(fluorosulfonyl) imide. Therefore, the electrolytic solution further enables the corresponding lithium-ion battery to have excellent quick charging performance and a long cycle life by containing the above type of lithium salt.

A second aspect of the present disclosure further provides a lithium-ion secondary battery. The electrolytic solution includes the electrolytic solution of the first aspect of the present disclosure. Therefore, the secondary battery has excellent quick charging performance and a long cycle life.

A third aspect of the present disclosure provides a battery module including the secondary battery of the second aspect of the present disclosure.

A fourth aspect of the present disclosure provides a battery pack including the battery module of the third aspect of the present disclosure.

A fifth aspect of the present disclosure provides a powered device, which includes at least one selected from the secondary battery of the second aspect of the present disclosure, the battery module of the third aspect of the present disclosure, or the battery pack of the fourth aspect of the present disclosure.

The electrolytic solution of the present disclosure contains carboxylic ester and fluorosulfonic acid lactone of Formula I at a specific proportion to adjust the compatibility of the carboxylic ester solvent and the negative electrode and further prevent reduction of the carboxylic ester in the electrolytic solution. According, the corresponding lithium-ion battery has excellent quick charging performance and a long cycle life.

### BRIEF DESCRIPTION OF DRAWINGS

FIG. 1 is a schematic diagram of a secondary battery according to an embodiment of the present disclosure.
FIG. 2 is an exploded view of a secondary battery according to the embodiment of the present disclosure shown in FIG. 1.
FIG. 3 is a schematic diagram of a battery module according to an embodiment of the present disclosure.
FIG. 4 is a schematic diagram of a battery pack according to an embodiment of the present disclosure.
FIG. 5 is an exploded view of a battery pack according to the embodiment of the present disclosure shown in FIG. 4.
FIG. 6 is a schematic diagram of a powered device using a secondary battery as a power supply according to an embodiment of the present disclosure.

### Reference signs:

1 battery pack; 2 upper box body; 3 lower box body; 4 battery module; 5 secondary battery; 51 housing; 52 electrode assembly; 53 top cover assembly.

### DESCRIPTION OF EMBODIMENTS

Embodiments of an electrolytic solution, a secondary battery, a battery module, a battery pack, and a powered device of the present disclosure are described in detail below with reference to the accompanying drawings. However, unnecessary detailed description may be omitted. For example, a detailed description of well-known items and a repeated description of an actually same structure are omitted. This is to avoid unnecessarily lengthy of the following description, thereby making it convenient for those skilled in the art to understand. In addition, the accompany drawings and the following description are provided to make those skilled in the art to fully understand the present disclosure, which is not intended to limit the subject matter recited in the claims thereto.

A "range" disclosed herein is defined in a form of a lower limit and an upper limit, and a given range is defined by selecting a lower limit and an upper limit, while the selected lower limit and upper limit define a boundary of a particular range. The range defined in this manner may include or do not include an end value and may be arbitrarily combined, that is, any lower limit may be combined with any upper limit to form a range. For example, if ranges of 60 to 120 and 80 to 110 are listed for a particular parameter, it is understood that ranges of 60 to 110 and 80 to 120 are also expected. Further, if minimum range values 1 and 2 are listed and maximum range values 3, 4, and 5 are listed, ranges of 1 to 3, 1 to 4, 1 to 5, 2 to 3, 2 to 4, and 2 to 5 may all be expected. In the present disclosure, unless otherwise stated, a numerical range "a to b" means an abbreviated representation of a combination of any real numbers between a to b, where a and b are both real numbers. For example, a numerical range "0 to 5" means that all real numbers between "0 and 5" are all listed herein, and "0 to 5" is merely an abbreviated representation of combinations of these numbers. In addition, when it is expressed that a certain parameter is an integer which is ≥ 2, it is equivalent to disclosing that the parameter is, for example, an integer 2, 3, 4, 5, 6, 7, 8, 9, 10, 11, 12, or the like.

If not specifically stated, all embodiments and optional embodiments of the present disclosure may be combined with each other to form a new technical solution.

If not specifically stated, all technical features and optional technical features of the present disclosure may be combined with each other to form a new technical solution.

If not specifically stated, all steps of the present disclosure may be performed sequentially or may be performed randomly, preferably sequentially. For example, the method includes steps (a) and (b), indicating that the method may include steps (a) and (b) performed sequentially or may include steps (b) and (a) performed sequentially. For example, the method may further include a step (c), indicating that the step (c) may be added to the method in any order, for example, the method may include steps (a), (b), and (c), or it may include steps (a), (c), and (b), or it may include steps (c), (a), and (b).

If not specifically stated, "include and "comprise" mentioned in the present disclosure represent an open type or may be a closed type. For example, the "include" and "comprise" may indicate that other un-listed components can be further included or comprised, or only listed components are included or comprised.

If not specifically stated, a term "or" is inclusive in the present disclosure. For example, a phrase "A or B" means "A, B, or both A and B". More specifically, the condition "A or B" is satisfied by either of the following conditions: A is true (or present) and B is false (or not present); A is false (or not present) and B is true (or present); or both A and B are true (or present).

The lithium iron phosphate (LFP) battery has long cycle life and high safety performance, but its low energy density becomes a fatal defect of the lithium iron phosphate (LFP) battery. In order to increase the energy density of the LFP battery, increasing a coating weight of a positive electrode material becomes a primary strategy of researchers. However, after increasing the coating weight, the thickness of the electrode plate is remarkably increased, and correspondingly, the transmission path of lithium ions is remarkably increased. Accordingly, it is difficult for the electrolytic solution of the common carbonate solvent system to meet quick charging requirement of the existing LFP battery with thick coating. The carboxylate solvent system is low in viscosity, and a conductivity of corresponding electrolytic solution is high. However, since compatibility of the carboxylate and the negative electrode is poor, the battery cell of the LFP system battery has extremely poor cycle and storage life. A first aspect of the present disclosure provides an electrolytic solution for a lithium-ion secondary battery. The electrolytic solution contains carboxylic ester and fluorosulfonic acid lactone with a specific structure, which can adjust the compatibility of the carboxylic ester solvent and the negative electrode and further prevent reduction of carboxylic ester in the electrolytic solution. Hence, the corresponding lithium-ion battery has an excellent quick charging performance and a long cycle life.

### Electrolytic Solution for Lithium-Ion Secondary Battery

In an embodiment of the present disclosure, the first aspect of the present disclosure provides an electrolytic solution for a lithium-ion secondary battery. The electrolytic solution contains solvent, additive, and lithium salt. The solvent includes carboxylic ester, and the additive includes fluorosulfonic acid lactone of Formula I as below 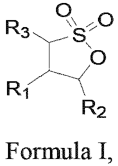 where R₁, R₂ and R₃ each are H or F independently, and at least one of R₁, R₂ and R₃ is F and at least one thereof is H.

In the electrolytic solution, the content W of the carboxylate ester and the content a of the fluorosulfonic acid lactone of Formula I satisfy 430.1 > 1000*a/W > 0.163, optionally satisfy 202.02 > 1000*a/w ≥ 1.635, and further optionally satisfy 100.059 ≥ 1000*a/w ≥ 4.915, where a and W are both in weight% based on the weight of the electrolytic solution.

In the present disclosure, the electrolytic solution contains carboxylic acid ester and fluorosulfonic acid lactone of Formula I in a specific proportion, such that the lithium salt anion is introduced into a solvation sheath layer of the lithium-ions, so as to form an anion-induced ion-solvent complex solvation structure (AI-ISC) having stronger reduction stability. Accordingly, the compatibility of the carboxylic ester solvent and the negative electrode can be adjusted. In addition, since the fluorosulfonic acid lactone has a relatively low electron cloud density, such that it is extremely easy to be electron-reduced at the negative electrode to form components of a compact solid electrolyte interface (SEI) film enriched with lithium alkyl sulfonate, or lithium alkyl sulfate or the like, thereby further preventing reduction of the carboxylic ester in the electrolytic solution. Therefore, the corresponding lithium-ion battery has excellent quick charging performance and a long cycle life.

In some embodiments, the content W of the carboxylic ester is 30-70 wt%. The content a of the fluorosulfonic acid lactone of Formula I is 0.01-10 wt%, optionally 0.5-5 wt%, further optionally 0.5-3 wt%, each of which is based on the weight of the electrolytic solution. Therefore, the electrolytic solution contains the carboxylic ester and the fluorosulfonic acid lactone of Formula I with the above contents, such that the corresponding lithium-ion battery has excellent quick charging performance and a long cycle life.

In some embodiments, the carboxylic ester is at least one selected from a group consisting of methyl formate (MF), methyl acetate (MA), ethyl acetate (EA), propyl acetate (PA), methyl propionate (MP), ethyl propionate (EP), and propyl propionate (PP). The fluorosulfonic acid lactone of Formula I is

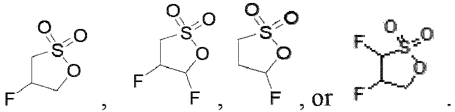

Therefore, the electrolytic solution contains the fluorosulfonic acid lactone of Formula I, type of which is described above, such that the corresponding lithium-ion battery has excellent quick charging performance and a long cycle life.

In some embodiments, in the electrolytic solution, the solvent is 70-90 wt%, preferably 80-88 wt%; the additive is 0.01-20 wt%, preferably 0.3-15 wt%; and the lithium salt is 10-22 wt%, preferably 10-15 wt%. A sum of wt% of the above components is 100% based on the weight of the electrolytic solution.

Therefore, the electrolytic solution contains the solvent, the additive, and the lithium salt with the above contents, such that the corresponding lithium-ion battery has excellent quick charging performance and a long cycle life.

In some embodiments, in the electrolytic solution, a weight ratio of the carboxylic ester in the solvent is 30%-80% based on the weight of the solvent, and a weight ratio of the fluorosulfonic acid lactone in the additive is 20-100 wt% based on the weight of the additive. Therefore, the electrolytic solution contains the carboxylic ester and the fluorosulfonic acid lactone of Formula I with the above contents, such that the corresponding lithium-ion battery has excellent quick charging performance and a long cycle life.

In some embodiments, the additive further includes O=C=N-R-N=C=O additive, where R is one of substituted or unsubstituted C1-C6 alkylene or alkyleneoxy, substituted or unsubstituted C2-C6 alkenylene or alkenyloxy, and substituted or unsubstituted C6-C12 arylene; preferably one of hexamethylene diisocyanate (HDI), toluene diisocyanate (TDI), isophorone diisocyanate (IPDI), diphenylmethane diisocyanate (MDI), and dicyclohexylmethane diisocyanate (H12MDI), further preferably hexamethylene diisocyanate or toluene diisocyanate (TDI). The toluene diisocyanate (TDI) includes 2,4-toluene diisocyanate and 2,6-toluene diisocyanate, preferably 2,4-toluene diisocyanate. Therefore, the electrolytic solution further enables the corresponding lithium-ion battery to have excellent quick charging performance and a long cycle life by containing the above types of isocyanate.

In some embodiments, the solvent further includes cyclic carbonate solvent selected from at least one of fluoroethylene carbonate (FEC), ethyl propyl carbonate (EC), propylene carbonate (PC), methyl ethyl carbonate (EMC), diethyl carbonate (DEC), dimethyl carbonate (DMC), propylene carbonate (DPC), methyl propyl carbonate (MPC), ethyl propyl carbonate (EPC), or butylene carbonate (BC), preferably one or more of ethyl propyl carbonate, propylene carbonate, and fluoroethylene carbonate. The content of the cyclic carbonate is 8.5%-35% of the electrolytic solution. Therefore, the electrolytic solution further enables the corresponding lithium-ion battery to have excellent quick charging performance and a long cycle life by containing the above types of solvent.

In some embodiments, the solvent includes one or more selected from a group consisting of methyl butyrate (MB), ethyl butyrate (EB), 1,4-butyrolactone (GBL), sulfolane (SF), dimethyl sulfone (MSM), methyl ethyl sulfone (EMS), and diethyl sulfone (ESE).

In some embodiments, the lithium salt is one or more selected from a group consisting of lithium hexafluorophosphate (LiPF₆), lithium tetrafluoroborate (LiBF₄), lithium perchlorate (LiClO₄), lithium hexafluoroarsenate (LiAsF₆), lithium bis(fluorosulfonyl) imide (LiFSI), lithium bis(trifluoromethanesulfonyl) imide (LiTFSI), lithium trifluoromethanesulfonate (LiTFS), lithium difluoro(oxalato)borate (LiDFOB), lithium bis(oxalate) borate (LiBOB), lithium difluorophosphate (LiPO₂F₂), lithium difluorobis(oxalato) phosphate (LiDFOP), and lithium tetrafluorooxalate phosphate (LiTFOP), optionally one or both of LiPF₆ or LiFSI. Therefore, the electrolytic solution further enables the corresponding lithium-ion battery to have excellent quick charging performance and a long cycle life by containing the above types of lithium salt.

In some embodiments, the additive includes a negative electrode film-forming additive and a positive electrode film-forming additive, and may further include an additive capable of improving certain properties of the battery, such as an additive for improving overcharge performance of the battery and an additive for improving high-temperature or low-temperature performance of the battery. The negative electrode film-forming additive is one or more selected from a group consisting of vinylene carbonate, fluoroethylene carbonate, and vinyl sulfate.

In some embodiments, the viscosity of the electrolytic solution is preferably < 3.5 mPa.s at 25°C, which is determined according to GB-T 10247-2008. The electrolytic solution has a conductivity that is > 12 mS cm at 25°C.

A second aspect of the present disclosure further provides a lithium-ion secondary battery including the electrolytic solution of the first aspect of the present disclosure. Therefore, the secondary battery has excellent quick charging performance and a long cycle life.

In addition, a secondary battery, a battery module, a battery pack, and a powered device of the present disclosure are described below with reference to the accompanying drawings.

In an embodiment of the present disclosure, a secondary battery is provided.

Generally, the secondary battery includes a positive electrode plate, a negative electrode plate, electrolyte, and an isolation film. In the charging and discharging processes of the battery, active ions are embedded and disengaged back and forth between the positive electrode plate and the negative electrode plate. The electrolyte acts as conductive ions between the positive electrode plate and the negative electrode plate. The isolation film is disposed between the positive electrode plate and the negative electrode plate, mainly preventing short-circuit of the positive and negative electrodes and allowing the ions to pass through.

### [Positive Electrode Plate]

The positive electrode plate includes a positive electrode current collector and a positive electrode film layer provided on at least one surface of the positive electrode current collector, and the positive electrode film layer includes the positive electrode active material of the first aspect of the present disclosure.

As an example, the positive electrode current collector has two surfaces opposite to each other in a thickness direction thereof, and the positive electrode film layer is disposed on any one or both of the two opposite surfaces of the positive electrode current collector.

In some embodiments, the positive electrode current collector may be a metal foil or a composite current collector. For example, the metal foil is an aluminum foil. The composite current collector may include a macromolecular material base-layer and a metal layer formed on at least one surface of the macromolecular material base-layer. The composite current collector may be formed by forming a metal material (such as, aluminum, aluminum alloy, nickel, nickel alloy, titanium, titanium alloy, silver, and silver alloy) on a macromolecular base material (such as, polypropylene (PP), polyethylene terephthalate (PET), polybutylene terephthalate (PBT), polystyrene (PS), and polyethylene (PE)).

In some embodiments, the positive electrode active material is a positive electrode active material for a battery known in the field. As an example, the positive electrode active material includes at least one of lithium-containing phosphate of an olivine structure, lithium transition metal oxide, or respective modified compounds thereof. However, the present disclosure is not limited to these materials, and other traditional materials that may be used as positive electrode active materials of the battery may be used. These positive electrode active materials may be used alone or in combinations of two or more. Examples of the lithium transition metal oxide may include, but are not limited to, at least one of lithium cobalt oxide (e.g., LiCoOz), lithium nickel oxide (e.g., LiNiOz), lithium manganese oxide (e.g., LiMnOz, LiMn₂O₄), lithium nickel cobalt oxide, lithium manganese cobalt oxide, lithium nickel manganese oxide, lithium nickel cobalt manganese oxide (e.g., LiNi_{1/3}Co_{1/3}Mn_{1/3}O₂, which may be referred to as NCM₃₃₃), LiNi_{0.5}Co_{0.2}Mn_{0.3}O₂ (which may be referred to as NCM₅₂₃), LiNi_{0.5}Co_{0.25}Mn_{0.25}O₂ (which may be referred to as NCM₂₁₁), LiNi_{0.6}Co_{0.2}Mn_{0.2}O₂ (which may be referred to as NCM₆₂₂), LiNi_{0.8}Co_{0.1}Mn_{0.1}O₂ (which may be referred to as NCM₈₁₁), lithium nickel cobalt aluminum oxide (e.g., LiNi_{0.85}Co_{0.15}Al_{0.05}O₂), or modified compound thereof. Examples of the lithium-containing phosphate of olivine structure may include, but are not limited to, at least one of lithium iron phosphate (such as LiFePO₄, which may be referred to as LFP), a composite material of lithium iron phosphate and carbon, lithium manganese phosphate (such as LiMnPO₄), a composite material of lithium manganese phosphate and carbon, lithium iron manganese phosphate, or a composite material of lithium iron manganese phosphate and carbon.

In some embodiments, the positive electrode film layer further optionally includes a binding agent. As an example, the binding agent may include at least one of polyvinylidene fluoride (PVDF), polytetrafluoroethylene (PTFE), vinylidene fluoride-tetrafluoroethylene-propylene terpolymer, vinylidene fluoride-hexafluoropropylene-tetrafluoroethylene terpolymer, tetrafluoroethylene-hexafluoropropylene copolymer, or fluorine-containing acrylate resin.

In some embodiments, the positive electrode film layer further optionally includes a conductive agent. As an example, the conductive agent may include at least one of superconducting carbon, acetylene black, carbon black, Ketjen black, carbon dots, carbon nanotubes, graphene, or carbon nanofibers.

In some embodiments, the positive electrode plate is prepared as follows. The above components for preparing the positive electrode plate, such as the positive electrode active material, the conductive agent, the binding agent and any other components, are dispersed in a solvent (e.g., N-methylpyrrolidone) to form positive electrode slurry. The positive electrode slurry is then coated on the positive electrode current collector, and the positive electrode plate is obtained after drying, cold pressing and other processes.

### [Negative Electrode Plate]

The negative electrode plate includes a negative electrode current collector and a negative electrode film layer disposed on at least one surface of the negative electrode current collector, and the negative electrode film layer includes a negative electrode active material.

As an example, the negative electrode current collector has two surfaces opposite to each other in a thickness direction thereof, and the negative electrode film layer is disposed on any one or both of the two opposite surfaces of the negative electrode current collector.

In some embodiments, the negative electrode current collector may be a metal foil or a composite current collector. For example, the metal foil is a copper foil. The composite current collector may include a macromolecular material base-layer and a metal layer formed on at least one surface of the macromolecular material base-material. The composite current collector may be formed by forming a metal material (such as, copper, copper alloy, nickel, nickel alloy, titanium, titanium alloy, silver, and silver alloy) on a macromolecular base material (such as, polypropylene (PP), polyethylene terephthalate (PET), polybutylene terephthalate (PBT), polystyrene (PS), and polyethylene (PE)).

In some embodiments, the negative electrode active material may be a negative electrode active material for a battery known in the field. As an example, the negative electrode active material may include at least one of artificial graphite, natural graphite, soft carbon, hard carbon, silicon-based material, tin-based material, or lithium titanate. The silicon-based material may be one or more selected from a group consisting of elemental silicon, silicon-oxygen compound, silicon-carbon composite, silicon-nitrogen compound, and silicon alloy. The tin-based material may be one or more selected from a group consisting of elemental tin, tin oxide compound, and tin alloy. However, the present disclosure is not limited to these materials, and other traditional materials that may be used as the negative electrode active materials of the battery may be used. These negative electrode active materials may be used alone or in combinations of two or more.

In some embodiments, the negative electrode active material has a volume average particle size Dv50 of 6 µm-14 µm, preferably 8 µm-12 µm. The negative electrode active material has a tap density of 0.9 g/cm³-1.15 g/cm³, preferably 0.9 g/cm³-1.05 g/cm³.

In some embodiments, the negative electrode film layer further optionally includes a binding agent. The binding agent may be one or more selected from a group consisting of styrene butadiene rubber (SBR), polyacrylic acid (PAA), sodium polyacrylate (PAAS), polyacrylamide (PAM), polyvinyl alcohol (PVA), sodium alginate (SA), polymethacrylic acid (PMAA), and carboxymethyl chitosan (CMCS).

In some embodiments, the negative electrode film layer further optionally includes a conductive agent. The conductive agent may be one or more selected from a group consisting of superconducting carbon, acetylene black, carbon black, Ketjen black, carbon dots, carbon nanotubes, graphene, and carbon nanofibers.

In some embodiments, the negative electrode film layer may optionally include other additives, such as a thickening agent, e.g., sodium carboxymethyl cellulose (CMC-Na).

In some embodiments, the negative electrode plate is prepared as follows. The above components for preparing the negative electrode plate, such as the negative electrode active material, the conductive agent, the binding agent and any other components, are dispersed in a solvent (for example, deionized water) to form negative electrode slurry. The negative electrode slurry is then coated on the negative electrode current collector, and the negative electrode plate is obtained after drying, cold pressing and other processes.

### [Electrolytic Solution]

The electrolytic solution functions to conduct ions between the positive electrode plate and the negative electrode plate.

In some embodiments, the electrolytic solution is the electrolytic solution in the first aspect of the present disclosure.

### [Isolation Film]

In some embodiments, the secondary battery further includes an isolation film. The type of the isolation film is not particularly limited in the present disclosure, and may be any well-known isolation film with a porous structure that has good chemical stability and mechanical stability.

In some embodiments, a material of the isolation film may be at least one selected from a group consisting of glass fiber, non-woven fabric, polyethylene, polypropylene, and polyvinylidene fluoride. The isolation film may be a single-layer film or may be a multilayer composite film, which is not particularly limited. When the isolation film is a multilayer composite film, materials of the layers may be the same or different, which is not particularly limited.

In some embodiments, the positive electrode plate, the negative electrode plate and the isolation film is made into an electrode assembly by means of a winding process or a lamination process.

In some embodiments, the secondary battery may include an outer package. The outer package is configured to package the above electrode assembly and the electrolyte.

In some embodiments, the outer package of the secondary battery may be a hard housing, such as a hard plastic housing, an aluminum housing, and a steel housing. The outer package of the secondary battery may be a soft package, such as a bag-type soft package. A material of the soft package may be plastic, such as polypropylene, polybutylene terephthalate, and polybutylene succinate.

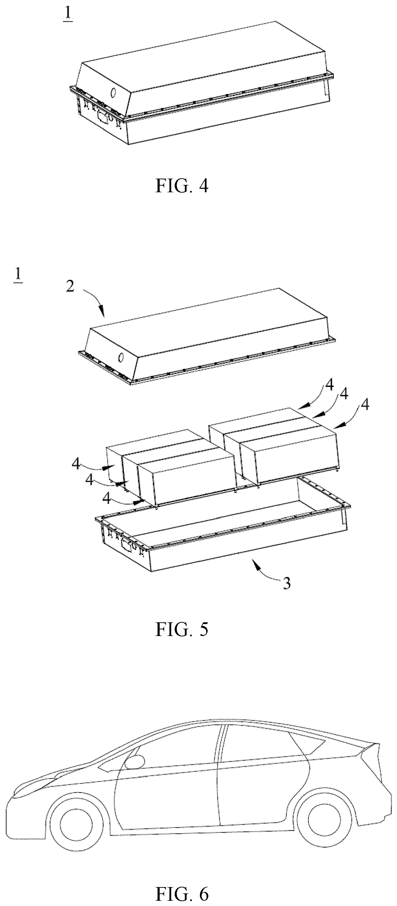

The shape of the secondary battery is not particularly limited in the present disclosure, and may be cylindrical, square, or any other shape. For example, FIG. 5 illustrates a secondary battery 5 having a square structure as an example.

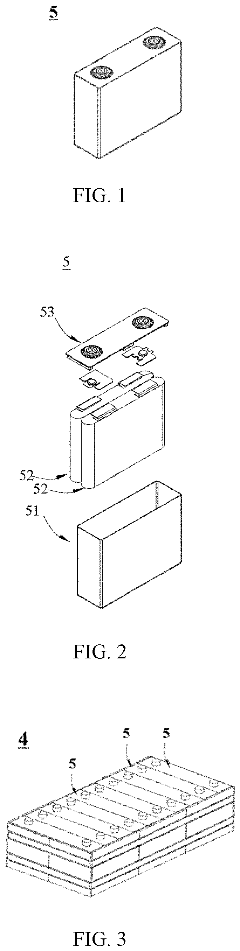

In some embodiments, referring to FIG. 2, the outer package includes a casing 51 and a cover plate 53. The casing 51 includes a bottom plate and a side plate connected to the bottom plate, and the bottom plate and the side plate enclose an accommodating cavity. The casing 51 has an opening in communication with the accommodating cavity, and the cover plate 53 can cover the opening to enclose the accommodating cavity. The positive electrode plate, the negative electrode plate and the isolation film forms an electrode assembly 52 through a winding process or a lamination process. The electrode assembly 52 is enclosed in the accommodating cavity. The electrolyte is immersed in the electrode assembly 52. The secondary battery 5 may include one or more electrode assemblies 52, and those skilled in the art can select according to specific actual requirements.

In some embodiments, the secondary battery may be assembled into a battery module, and the battery module may include one or more secondary batteries, the specific number of which can be selected by those skilled in the art according to the application and a capacity of the battery module.

FIG. 3 illustrates a battery module 4 as an example. Referring to FIG. 3, a plurality of secondary batteries 5 of the battery module 4 is sequentially arranged along a length direction of the battery module 4. Alternatively, the secondary batteries may be arranged in any other manner. The plurality of secondary batteries 5 can be further fixed by fasteners.

Optionally, the battery module 4 may further include a shell having an accommodating space, and the plurality of secondary batteries 5 is accommodated in the accommodating space.

In some embodiments, the above battery modules may be assembled into a battery pack, and the number of the battery modules contained in the battery pack may be one or more, specific number of which can be selected by those skilled in the art according to the application and capacity of the battery pack.

FIG. 4 and FIG. 5 show a battery pack 1 as an example. Referring to FIG. 4 and FIG. 5, the battery pack 1 includes a battery box and a plurality of battery modules 4 provided in the battery box. The battery box includes an upper box body 2 and a lower box body 3, and the upper box body 2 can cover the lower box body 3 to form a closed space for accommodating the battery module 4. The plurality of battery modules 4 may be arranged in the battery box in any manner.

In addition, the present disclosure further provides a powered device, and the powered device includes at least one of the secondary battery, the battery module, or the battery pack according to the present disclosure. The secondary battery, the battery module or the battery pack may be used as a power supply for the powered device, or may be used as an energy storage unit of the powered device. The powered device may include a mobile device (such as a mobile phone and a notebook computer), an electric vehicle (such as a pure electric vehicle, a hybrid electric vehicle, a plug-in hybrid electric vehicle, an electric bicycle, an electric scooter, an electric golf cart, and an electric truck), an electrical train, a ship, a satellite, an energy storage system and the like, but it is not limited thereto.

The powered device may select the secondary battery, the battery module, or the battery pack according to use requirements thereof.

FIG. 6 illustrates a powered device as an example. The powered device is a pure electric vehicle, a hybrid electric vehicle, a plug-in hybrid electric vehicle and the like. In order to meet requirements of the powered device for high power and high energy density of the secondary battery, a battery pack or a battery module can be adopted.

As another example, a device may be a mobile phone, a tablet computer, a notebook computer and the like. The device is usually required to be light and thin, and the secondary battery can be used as a power supply.

### Embodiments

In order to make the technical problems to be solved, the technical solutions and the beneficial effects clearer, the present disclosure will be further described in detail below in combination with embodiments and the drawings. It is apparent that the described embodiments are only a part of the embodiments of the present disclosure, rather than all embodiments. The following description of at least one exemplary embodiment is merely illustrative, and is not intended as limitation on the present disclosure and its application. Based on the embodiments of the present disclosure, all other embodiments obtained by those skilled in the art without creative efforts shall fall within the scope of the present disclosure.

Specific techniques or conditions, which are not specified in the embodiments, are carried out in accordance with techniques or conditions described in literature in the art or in accordance with a specification of a product. The reagents or instruments used without any indication of manufacturers are conventional products available commercially.

### Embodiment 1

### 1) Preparation of Positive Electrode Plate

The positive electrode active material of lithium iron phosphate, the conductive agent of acetylene black and the binding agent of polyvinylidene fluoride (PVDF) are dissolved in the solvent of N-methyl pyrrolidone (NMP) at a weight ratio of 96.5:1.5:2, and are fully stirred and uniformly mixed to obtain positive electrode slurry. After that, the positive electrode slurry is coated uniformly on the positive electrode current collector, and is then dried, cold-pressed, and slit to obtain the positive electrode plate. A loading capacity of the positive electrode active material on one side of the positive electrode current collector is 0.0263 g/cm².

### 2) Preparation of Negative Electrode Plate

The active material of artificial graphite, the conductive agent of acetylene black, the binding agent of styrene-butadiene rubber (SBR) and the thickening agent of sodium carboxymethyl cellulose (CMC) are dissolved in the solvent of deionized water at a weight ratio of 95:2:2:1 and uniformly mixed with the solvent of deionized water, to prepare negative electrode slurry. After that, the negative electrode slurry is coated uniformly on a copper foil of the negative electrode current collector, and is then dried to obtain a negative electrode film. The negative electrode film is then cold-pressed and slit to obtain a negative electrode plate. A loading capacity of the negative electrode active material on one side of the negative electrode current collector is 0.0121 g/cm².

### 3) Isolation Film

A polypropylene film is used as an isolation film.

### 4) Preparation of Electrolytic Solution

In an argon atmosphere glove box (H₂O < 0.1 ppm, O₂ < 0.1 ppm), the components of the electrolytic solution listed in the table are stirred to be mixed uniformly to obtain the corresponding electrolytic solution.

### 5) Preparation of Battery

The positive electron plate, the isolation film and the negative electron plate are stacked in sequence, such that the isolation film is arranged between the positive electron plate and the negative electron plate to isolate them. The positive electron plate, the isolation film and the negative electron plate are then wound to obtain an electrode assembly The electrode assembly is then placed in a battery housing and dried, and after that, the electrolyte is injected in the battery housing and then processes such as chemical formation and standing are performed to obtain a lithium ion battery.

Preparation methods of the secondary batteries of Embodiments 2-17 and the secondary batteries of Comparative Examples 1-2 are similar to that of the secondary battery of Embodiment 1. However, compositions and parameters of the additive, the solvent and lithium salt in the electrolytic solution are adjusted, referring to Table 1 for details.

**Table 1 Compositions and Related Parameters of Electrolytic Solution of Embodiments and Comparative Examples**

| Serial Number of Embodiments | Solvent | Additive | | Lithium Salt | | Content and Whether Isocyanate is Contained | Relation between Fluorosulfonic Acid Lactone a and Carboxylate W (1000*a/W) |
|---|---|---|---|---|---|---|---|
| | Type and Ratio of Weight | Type | Content % | Type | WT% | | |
| 1 | EA/DMC/PA =2/1/7 | 3-fluoro-1,3-propanesulf onic acid lactone | 1% | LiPF6 | 12.5 | NO | 16.515 |
| 2 | EA/DMC /PA =2/1/7 | 2-fluoro-1,3-propanesulf onic acid lactone | 1 | LiPF6 | 12.5 | NO | 16.515 |
| 3 | EA/DMC /PA =2/1/7 | 2,3-difluoro-1,3-propanesulf onic acid lactone | 1 | LiPF6 | 12.5 | NO | 16.515 |
| 4 | EA/DMC /PA =2/1/7 | 1,2-difluoro-1,3-propanesulf onic acid lactone | 1 | LiPF6 | 12.5 | NO | 16.515 |
| 5 | EA/DMC /PP =2/1/7 | 3-fluoro-1,3-propanesulf onic acid lactone | 1 | LiPF6 | 12.5 | NO | 16.515 |
| 6 | EA/DMC/MA /EA =2/1/2/2/3 | 3-fluoro-1,3-propanesulf onic acid lactone | 1 | LiPF6 | 12.5 | NO | 16.515 |
| 7 | EA/DMC /PA =1/1/8 | 3-fluoro-1,3-propanesulf onic acid lactone | 0.01 | LiPF6 | 12.5 | NO | 0.143 |
| 8 | EA/DMC /PA =2/1/7 | 3-fluoro-1,3-propanesulf onic acid lactone | 0.01 | LiPF6 | 12.5 | NO | 0.163 |
| 9 | EA/DMC /PA =2/1/7 | 3-fluoro-1,3-propanesulf onic acid lactone | 0.1 | LiPF6 | 12.5 | NO | 1.635 |
| 10 | EA/DMC /PA =2/1/7 | 3-fluoro-1,3-propanesulf onic acid lactone | 0.3 | LiPF6 | 12.5 | NO | 4.915 |
| 11 | EA/DMC /PA =2/5/3 | 3-fluoro-1,3-propanesulf onic acid lactone | 2 | LiPF6 | 12.5 | NO | 100.059 |
| 12 | EA/DMC /PA =2/5/3 | 3-fluoro-1,3-propanesulf onic acid lactone | 5 | LiPF6 | 12.5 | NO | 202.02 |
| 13 | EA/DMC /PA =2/5/3 | 3-fluoro-1,3-propanesulf onic acid lactone | 10 | LiPF6 | 12.5 | NO | 430.108 |
| 14 | EA/DMC /PA =2/5/3 | 3-fluoro-1,3-propanesulf onic acid lactone | 12 | LiPF6 | 12.5 | NO | 529.801 |
| 15 | EA/DMC /PA =2/1/7 | 3-fluoro-1,3-propanesulf onic acid lactone + 2-fluoro-1,3-propanesulf onic acid lactone | 0.5+0.5 | LiPF6 | 12.5 | NO | 16.515 |
| 16 | EA/DMC /PA =2/1/7 | 3-fluoro-1,3-propanesulf onic acid lactone | 1 | LiFSI+ LiPF6 | 10+5 | NO | 16.515 |
| 17 | EA/DMC /PA =2/1/7 | 3-fluoro-1,3-propanesulf onic acid lactone + 2-fluoro-1,3-propanesulf onic acid lactone | 0.5+0.5 | LiPF6 | 12.5 | hexamethylene diisocyanate 1% | 16.515 |
| 18 | EA/DMC /PA =2/1/7 | 3-fluoro-1,3-propanesulf onic acid lactone + 2-fluoro-1,3-propanesulf onic acid lactone | 0.5+0.5 | LiPF6 | 12.5 | 2,4-toluene diisocyanate 1% | 16.515 |
| Comparative Example 1 | EA/DMC=2/8 | None | | LiPF6 | 12.5 | NO | N/A |
| Comparative Example 2 | EA/DMC /PA =2/1/7 | None | | LiPF6 | 12.5 | NO | N/A |
| Comparative Example 3 | EA/DMC /PA =2/1 /7 | 1,2,3-trifluoro-1,3-propanesulf onic acid lactone | 1 | LiPF6 | 12.5 | NO | 16.515 |

It is noted that contents of the additive, lithium salt and isocyanate are all measured based on the weight of the electrolytic solution.

### Second. Test of Battery Performance

### 1. Test of Cycle Performance of Lithium-ion Battery at 45 °C

At 45°C, the lithium-ion battery is charged to 3.65 V at a constant current of 1 C, then charged at a constant voltage of 3.65 V until the current is less than 0.05 C, and thereafter discharged to 2.5 V at a constant current of 1 C, to obtain a first discharging capacity of C0 that is recorded as a first cycle. In this way, charging and discharging are repeatedly carried out, and the number of cycles during which the discharging capacity of the lithium-ion battery degrades to 80% of C0 is calculated.

### 2. Test of Rapid Charging Capability of Battery

At 25 °C, three-electrode secondary batteries prepared according to the Embodiments and the Comparative Examples are charged at a constant current of 0.33 C until a charging cut-off voltage is 3.65 V, that is, a current value of a theoretical capacity is completely released in one hour. After that, the secondary batteries are charged at a constant voltage until the current is 0.05 C, and stands for 5 min. The secondary batteries are then discharged at a constant current of 0.33 C until a discharging cut-off voltage is 2.5 V, and an actual capacity thereof is recorded as C0.

Thereafter, the battery is charged sequentially at a constant current of 0.5C0, 1C0, 1_5C0, 2C0, 2.5C0, 3C0, 3_5C0, 4C0, and 4.5C0 until a full-battery charging cut-off voltage 3.65 V or a 0V negative electrode cut-off potential (taking the one achieved first as standard). After each charging is completed, the battery is required to be discharged at 1C0 to a full-battery discharge cut-off voltage of 2.5 V, and respective negative electrode potentials when being charged to 10%SOC, 20%SOC, 30%SOC,......, 80%SOC at different charging rates are recorded. Rate-negative electrode potential curves at different SOC states are drawn, and after linear fitting, respective charging rates when the negative electrode potential is 0V at different SOC states are obtained. The charging rate is a charging window at this SOC state. The charging windows at 20%SOC, 30%SOC,......, 80%SOC are respectively marked as C20%SOC, C30%SOC, C40%SOC, C50%SOC, C60%SOC, C70%SOC, and C80%SOC. Charging time T (min) of the battery charged from 10%SOC to 80%SOC is obtained by calculating according to a formula (60/C/20%SOC+60/C30%SOC +60/C40%SOC+60/C50%SOC+60/C60%SOC+60/C70%SOC+60/C80%SOC)×10%. The shorter the time, the better the rapid charging capability of the battery is.

### Third. Test Results of Embodiments and Comparative Examples

The batteries in various Embodiments and Comparative Examples are prepared respectively according to the above methods, and various performance parameters are tested. The results are shown in Table 2 below.

**Table 2 Battery Properties of Embodiments and Comparative Examples**

| Serial Number of Embodiments | Cycle Life at 45°C cls | 0-80%SOC Quick Charging Time/min |
|---|---|---|
| 1 | 1032 | 15.5 |
| 2 | 1089 | 16 |
| 3 | 1102 | 16.5 |
| 4 | 1008 | 15.8 |
| 5 | 1084 | 15.2 |
| 6 | 1067 | 15 |
| 7 | 398 | 13.6 |
| 8 | 498 | 15.1 |
| 9 | 835 | 15 |
| 10 | 912 | 15.3 |
| 11 | 1067 | 15.5 |
| 12 | 1021 | 17.3 |
| 13 | 632 | 20.2 |
| 14 | 547 | 25 |
| 15 | 1164 | 15.7 |
| 16 | 1198 | 14.8 |
| 17 | 1287 | 15.8 |
| 18 | 1256 | 16.3 |
| Comparative Example 1 | 312 | 52 |
| Comparative Example 2 | 202 | 18.3 |
| Comparative Example 3 | 443 | 17.9 |

It can be seen from Table 1 and Table 2 that the electrolytic solution of the present disclosure enables a corresponding battery to have excellent quick charging performance and a long cycle life. For example, 0-80%SOC quick charging time is reduced to about 15 min under the cycle life with at least 1100 cycles.

It should be noted that the present disclosure is not limited to the above-mentioned embodiments. The above embodiments are merely examples, and embodiments having substantially the same composition as that of technical ideas and playing the same effect in the range of the technical solution of the present disclosure are all included in the technical scope of the present disclosure. In addition, without departing from the spirit of the present disclosure, various modifications which are applied to the embodiments and can be conceived by those skilled in the art, and other manners constructed by combining part of the components in the embodiments are also included within the scope of the present disclosure.

## Claims

1. An electrolytic solution for a lithium-ion secondary battery, comprising solvent, additive, and lithium salt,
wherein the solvent comprises carboxylic ester, and the additive comprises fluorosulfonic acid lactone of Formula I as below 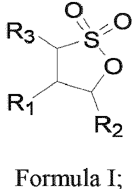
wherein R₁, R₂ and R₃ each are H or F independently, and at least one of R₁, R₂ and R₃ is F and at least one other of R₁, R₂ and R₃ is H;
wherein in the electrolytic solution, a content W of carboxylic ester and a content a of fluorosulfonic acid lactone of Formula I satisfy 430.1 > 1000*a/W > 0.163, optionally 202.02 > 1000*a/w ≥ 1.635, and further optionally 100.059 ≥ 1000*a/w ≥ 4.915; and
wherein a and W are both in wt% based on a weight of the electrolytic solution.

2. The electrolytic solution according to claim 1, wherein the content W of carboxylic ester is 30 wt% to 70 wt%, and the content a of fluorosulfonic acid lactone of Formula I is 0.01 wt% to 10 wt%, each based on the weight of the electrolytic solution.

3. The electrolytic solution according to claim 1 or 2, wherein the carboxylic ester is at least one selected from methyl formate, methyl acetate, ethyl acetate, propyl acetate, methyl propionate, ethyl propionate, and propyl propionate, and fluorosulfonic acid lactone of Formula I is

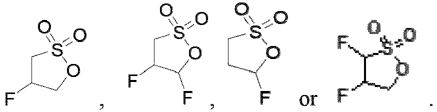

4. The electrolytic solution according to any one of claims 1-3, wherein in the electrolytic solution,
the solvent is 70 wt% to 90 wt%;
the additive is 0.01 wt% to 20 wt%;
the lithium salt is 10 wt% to 22 wt%; and
a sum of weight percentages of the solvent, the additive and the lithium salt is 100% based on the weight of the electrolytic solution.

5. The electrolytic solution according to any one of claims 1-4, wherein in the electrolytic solution, a weight percentage of carboxylic ester in the solvent is 30% to 80% based on a weight of the solvent, and a weight percentage of the fluorosulfonic acid lactone in the additive is 20 wt% to 100 wt% based on a weight of the additive.

6. The electrolytic solution according to any one of claims 1-5, wherein the additive further comprises O=C=N-R-N=C=O additive, where R is one of substituted or unsubstituted C1-C6 alkylene or alkyleneoxy, substituted or unsubstituted C2-C6 alkenylene or alkenyloxy, and substituted or unsubstituted C6-C12 arylene; optionally at least one of hexamethylene diisocyanate (HDI), toluene diisocyanate (TDI), isophorone diisocyanate (IPDI), diphenylmethane diisocyanate (MDI), and dicyclohexylmethane diisocyanate (H12MDI); and further optionally hexamethylene diisocyanate or toluene diisocyanate.

7. The electrolytic solution according to any one of claims 1-6, wherein the solvent further comprises a solvent of cyclic carbonate, and the cyclic carbonate is optionally one or more of ethyl propyl carbonate, propylene carbonate, and fluoroethylene carbonate; and
a content of the cyclic carbonate is 8.5% to 35% of the electrolytic solution.

8. The electrolytic solution according to any one of claims 1-7, wherein the lithium salt is one or more selected from lithium hexafluorophosphate, lithium tetrafluoroborate, lithium perchlorate, lithium hexafluoroarsenate, lithium bis(fluorosulfonyl) imide, lithium bis(trifluoromethanesulfonyl) imide, lithium trifluoromethanesulfonate, lithium difluoro(oxalato)borate, lithium bis(oxalate) borate, lithium difluorophosphate, lithium difluorobis(oxalato) phosphate, and lithium tetrafluorooxalate phosphate, optionally one or both of lithium hexafluorophosphate or lithium bis(fluorosulfonyl) imide.

9. A lithium-ion secondary battery, comprising the electrolytic solution according to any one of claims 1-8.

10. A battery module, comprising the secondary battery according to claim 9.

11. A battery pack, comprising the battery module according to claim 10.

12. A powered device, comprising at least one selected from the secondary battery according to claim 9, the battery module according to claim 10, or the battery pack according to claim 11.
